# EUROPEAN PATENT APPLICATION

(11) **EP 0 555 717 A1**
(43) Date of publication of application: **18.08.1993**
(21) Application number: 93101501.0
(22) Date of filing: 10.12.1987
(51) Int. Cl.: A61K 31/47

(54) **Pharmaceutical compositions in the form of solutions containing nedocromil**

(30) Priority: 23.12.1986 GB 8630767; 23.12.1986 GB 8630769; 24.12.1986 GB 8630904; 20.03.1987 GB 8706684
(62) Divisional of application: 87310882.3
(71) Applicant: FISONS plc, Ipswich Suffolk IP1 1QH (GB)
(72) Inventor: Clark, Andrew Reginald, Half Moon Bay, CA 94019 (US); Ratcliffe, Julia Helena, Alverstoke, Gosport, Hampshire PO12 2DH (GB); Wright, Paul, Bramcote, Nottingham NG9 3FS (GB)
(74) Representative: Jones, Stephen Anthony

(57) **Abstract**

Pharmaceutical compositions comprising an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, the solution having a pH of from 3.5 to 6, are disclosed.

Also described are methods for their manufacture and their use in the treatment of e.g. conjunctivitis, keratitis, 'allergic eyes', adenovirus infections, corneal homograft rejection, anterior uveitis, nasal polyps, vasomotor rhinitis, allergic manifestations of the nasopharynx, reversible obstructive airways disease, Crohn's disease, distal colitis and proctitis.

## Description

This invention relates to novel pharmaceutical compositions, methods for their preparation and methods of treatment using them.

In British Patent No. 2022078 there are disclosed a number of pyranoquinoline compounds which are indicated for use in the treatment of reversible airway obstruction. Pharmaceutical compositions containing these compounds are also described, especially compositions in which the active ingredient is in powder form with a mass median diameter of from 0.1 to 10 µm. British Patent Application No. 2157291A describes the particular utility of the disodium salt of one of these compounds, 9-ethyl-6,9-dihydro-4,6-dioxo-10-propyl-4H-pyrano(3,2-g)quinoline-2,8-dicarboxylic acid, in the treatment of reversible airway obstruction. Also described are powdered aerosol compositions of this salt for administration to the lung and physical forms of the salt which are especially suitable for formulation in this way.

We have now surprisingly found that when it is administered in aqueous solution the same compound is efficacious in the treatment of a variety of disorders of the eye, notably conjunctivitis, as well as in the treatment of certain disorders associated with other organs.

Thus, according to the invention there is provided a pharmaceutical composition comprising an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, the solution having a pH of from 3.5 to 6.

Pharmaceutically acceptable salts of the active ingredient include salts with metal cations, such as alkali metal cations. We particularly prefer the disodium salt which is commonly referred to as nedocromil sodium.

The solutions according to the invention will be administered by a route appropriate to the condition being treated. For instance, administration may be to the eye, intra-nasally (e.g. as a nasal spray), by inhalation as a nebulised cloud or rectally as an enema.

The solutions may be used for the treatment of the following conditions:
conjunctivitis, keratitis, 'allergic eyes', adenovirus infections, corneal homograft rejection, anterior uveitis;
nasal polyps, vasomotor rhinitis, allergic manifestations of the nasopharynx;
reversible obstructive airways disease;
Crohn's disease, distal colitis and proctitis.

By the term 'conjunctivitis' we mean inflammatory disorders of the conjunctiva commonly characterised by photophobia and irritation. The condition may be bacterial or viral and encompasses a number of specific types of conjunctivitis; for instance, seasonal allergic conjunctivitis, perennial allergic conjunctivitis, vernal catarrh (vernal kerato-conjunctivitis), 'irritable eye' or 'non-specific conjunctivitis', Herpes Simplex Conjunctivitis, Herpes Zoster Conjunctivitis and phlyctenular conjunctivitis.

Similarly, by the term 'keratitis' we mean inflammation of the cornea which may involve its superficial surface ('superficial keratitis' including the localised form known as 'corneal ulceration') or be confined to the deeper layers ('interstitial keratitis'). Other particular forms of keratitis which may be mentioned include Herpes Simplex Keratitis and Herpes Zoster Keratitis.

Proctitis includes chronic (i.e. ulcerative) and non-specific proctitis.

The solution may contain from 0.1 to 10% w/v of the active ingredient. However, we prefer the active ingredient to be present at a level of less than 5% and more particularly less than 3% w/v, e.g. 0.5%, 1.0% or 2.0% w/v. The concentration of choice will depend of course inter alia on the nature of the condition to be treated, its severity and the mode of administration of the solution.

In addition to the active ingredient the solution generally contains one or more pharmaceutically acceptable additives. Examples of classes of additive which may be present are:
a) chelating or sequestering agents,
b) preservatives, and
c) viscosity-modifying agents.

Suitable chelating or sequestering agents include sodium carboxymethyl cellulose, citric, tartaric or phosphoric acid, and amino carboxylate compounds. The preferred chelating agent, however, is ethylenediamine tetraacetic acid or a salt thereof, especially its di-sodium salt.

The concentration of the chelating or sequestering agent should be such as to ensure that no precipitate of metal salts of the active ingredient occurs. A suitable concentration of chelating or sequestering agent may be from 0.005 to 0.5, e.g. 0.01 or 0.1% w/v.

The choice of preservative, where the solution contains a preservative, may depend on the route of administration. Preservatives suitable for solutions to be administered by one route may possess detrimental properties which preclude their administration by another route. For nasal and ophthalmic solutions, preferred preservatives include quaternary ammonium compounds, in particular the mixture of alkyl benzyl dimethyl ammonium compounds known generically as 'Benzalkonium Chloride'. For solutions to be administered by inhalation, however, the preferred preservative is chlorbutol. Other preservatives which may be used, especially for solutions to be administered rectally, include alkyl esters of p-hydroxybenzoic acid and mixtures thereof, such as the mixture of the methyl, ethyl, propyl and butyl esters sold under the name Nipastat .

The concentration of preservative should be such as to ensure effective preservation of the solution i.e. such that bacterial growth in the solution is inhibited. For most preservatives the concentration will typically lie in the range 0.001 to 0.1% w/v. However, in the case of chlorbutol acceptable concentrations are greater than 0.25% but less than 0.6% w/w, i.e. the concentration of chlorbutol is 0.25 to 0.6%, preferably 0.3 to 0.55%, e.g. 0.5% w/w.

Suitable viscosity enhancing agents which may be incorporated into the solution include carbomers i.e. polymers of acrylic acid cross-linked with a polyalkenyl polyether, aluminium magnesium silicate, methylcellulose, hydroxypropyl methylcellulose and other cellulose derivatives.

The viscosity of the solution will depend *inter alia* on the particular viscosity enhancing agent used and its molecular weight and on the target organ. However, the solution preferably has a viscosity of at least 100 mPa.s, more preferably at least 200 mPa.s and especially more than 400 mPa.s, at a shear rate of 50 s⁻¹. The viscosity of the solution is preferably less than 10000 mPa.s, more preferably less than 1000 mPa.s and especially less than 500 mPa.s, at a shear rate of 50 s⁻¹.

Viscosities are preferably determined using a rotational viscometer such as a Rheomat 30, at a temperature of from 15 to 25°C, e.g. 20°C.

For applications which involve the solution being administered as a spray eg through a nasal pump, we prefer the viscosity enhancing agent to have a low viscosity at high shear rates, e.g. from 100 mPa.s to 300 mPa.s at 140 s⁻¹, and a high viscosity at low shear rates, e.g. from 700 mPa.s to 1200 mPa.s at 15 s⁻¹.

Viscosity enhancing agents which we prefer include hydroxypropyl methylcellulose and carbomers, in particular the carbomer sold as Carbopol 934P, the viscosity of a neutralised 0.5% w/w aqueous dispersion of which lies in the range 29400 to 39400 mPa.s and the heavy metal content of which is 0.002% or less.

The amount of viscosity-modifying agent required to achieve the desired viscosity will depend on the particular agent used and also on its molecular weight. However, we prefer to use up to about 2% w/w, e.g. 0.5 to 1.5% w/w, of viscosity enhancing agent.

The solution may also contain other conventional excipients, e.g. sodium chloride, dextrose or mannitol, and buffers, e.g. sodium dihydrogen orthophosphate (sodium acid phosphate BP), di-sodium hydrogen phosphate (sodium phosphate BP) sodium citrate/citric acid, and boric acid/sodium borate. The proportion and concentration of excipients and buffers may be varied within fairly wide ranges, provided the resulting solution is stable and non-irritant when applied to the appropriate tissues. The maximum total concentration of excipients and buffers is preferably less than 5% w/v and more preferably less than 2% w/v. Solutions for rectal administration may contain bulking agents, e.g. methyl cellulose, to aid retention in the bowel.

The solution may be made isotonic with physiological fluids by the incorporation of a suitable tonicity agent e.g. sodium chloride. The solution typically contains from about 0.1 to 1.0, more typically 0.5 to 1.0% w/v, sodium chloride.

Although physiological pH is about 7.4, the pH of the solution is in the range 3.5 to 6, preferably 4 to 5.5. In this range of pH, the stability of the solution is enhanced, surprisingly with no deleterious effects such as undue tissue irritation.

The composition of the invention may be made up, for example, by dissolving the active ingredient, chelating or sequestering agent (if included) and excipients (if included) in freshly distilled water, adding to this solution an aqueous solution containing the preservative (if included), adjusting the pH if necessary, making the solution up to the desired volume with distilled water, stirring and then sterilising. Alternatively, the active ingredient, chelating or sequestering agent (if included) and excipients (if included) may be dissolved in a solution containing the preservative. Sterilisation is preferably performed by sterile filtration into a previously sterilised container. Where the preservative used is benzalkonium chloride, some complex formation may occur when the solutions of active ingredient and preservative are mixed. It may thus be necessary to use a higher concentration of preservative than is desired for the final product.

Aqueous solutions containing active ingredient, a viscosity enhancing agent, e.g. a carbomer, and, optionally, a preservative, e.g. benzalkonium chloride, may be prepared by dispersing the viscosity enhancing agent in an aqueous solution containing the preservative (if used) and the active ingredient, and then, if required, adjusting the pH, e.g. by addition of sodium hydroxide, and, if desired, further diluting the solution with water.

The solution of the preservative and the active ingredient may be made simply by dissolving the ingredients in water which is low in metal ions.

The solution is preferably made up at a temperature of from 10 to 50°C, for example at room temperature.

The solution may be put up in unit dosage form, in which case preservatives may be incorporated, but are generally not necessary. Alternatively the solution may be put up in multi-dose form. In general it will be necessary to incorporate one or more preservatives into multi-dose solutions to ensure that the solution remains sterile after initial use.

Multi-dose solutions may be packaged in volumes of 5 to 300 ml. Preferred volumes for inhalation compositions include 60, 120 and 240 ml. For nasal and ophthalmic compositions multi-dose packs preferably contain from 5 to 20 ml of solution.

We prefer multi-dose solutions to be packaged such that unit volumes of the solution to be administered can be accurately dispensed. The solution may, for example, be packaged in a flexible-walled container provided with a cap to receive the unit volume.

The dosage to be administered will of course vary with the condition to be treated, with its severity and with its location. However, in general for use in the eye a dosage of 1 or 2 drops (e.g. from 0.3 to 1.2 mg of active ingredient depending on the concentration of active ingredient) into the affected eye from 2 to 4 times a day is found to be satisfactory. More frequent dosage may, of course, be used if desired. For use in the nose a dosage of about 0.25 ml (e.g. from 1.2 mg to 5.0 mg of active ingredient) is indicated.

For rectal administration a total daily dosage of from 50 to 1000 mg of active ingredient, more preferably 100 to 400 mg, administered in smaller doses 2 to 4 times a day is found to be satisfactory. A dosage unit may conveniently contain from about 25 to 200 mg of active ingredient.

For administration by inhalation a daily dosage of from 10 mg to 100 mg is, in general, found to be satisfactory. The daily dosage may be administered in divided doses, e.g. from 2 to 4 times a day. More frequent dosage may of course be used if required.

The aqueous solutions according to the present invention are advantageous in that they are longer acting, more acceptable to the patient, give rise to higher concentrations of active ingredient in target tissues, give rise to effective concentrations of active ingredient in target tissues for a longer time or are more stable than known similar formulations.

The invention is illustrated by the following Examples.

### Example 1

### Non-preserved nebuliser solution

| | |
|---|---|
| Nedocromil Sodium | 0.5 % w/v |
| Sodium Chloride | 0.79 |
| Hydrochloric acid | q.s. |
| Purified Water | to 100 |

Nedocromil sodium (5 g) and sodium chloride (7.9 g) were dissolved in purified water (900 ml). The pH of the solution was adjusted to between 5 and 5.5 by addition of hydrochloric acid and the volume made up to 1000 ml with purified water.

The solution was sterile-filled into low-density polyethylene ampoules which were then sealed.

### Example 2

### Preserved nebuliser solution

| | |
|---|---|
| Nedocromil Sodium | 0.5 % w/v |
| Sodium Chloride | 0.79 |
| Chlorbutol | 0.5 |
| Sodium hydroxide | q.s. |
| Purified Water | to 100 |

Chlorbutol (5 g) was dissolved in purified water (900 ml). Nedocromil sodium (5 g) and sodium chloride (7.9 g) were then added to the solution. The pH of the solution was adjusted to between 5 and 5.5 by addition of sodium hydroxide and the volume made up to 1000 ml with purified water.

The solution was filled into polyethylene bottles of 120 ml capacity.

### Example 3

### Nasal Solution

| | |
|---|---|
| Nedocromil Sodium | 1.00 % w/v |
| Sodium Chloride | 0.715 |
| Disodium Edetate | 0.01 |
| Benzalkonium Chloride | 0.02 |
| Purified Water | to 100 |

Nedocromil sodium (100 g), sodium chloride (71.5 g) and disodium edetate (1 g) were dissolved in approximately 5 litres of purified water. To this solution a dispersion of benzalkonium chloride solution 50% USNF (4 g) in approximately 1 litre of purified water was added. The solution was made up to 10 litres with purified water, stirred for 30 minutes, filtered to remove any complex formed and then sterile filtered and filled into bottles.

### Example 4

### Ophthalmic Solution

| | |
|---|---|
| Nedocromil Sodium | 2.00 % w/v |
| Benzalkonium Chloride | 0.01 |
| Disodium Edetate | 0.05 |
| Sodium Chloride | 0.55 |
| Purified Water | to 100 |

Prepared by the method of Example 3 above.

### Example 5

### Viscous Nasal or Ophthalmic Solution

| | |
|---|---|
| Nedocromil sodium | 1.0 % w/w |
| Disodium edetate | 0.1 |
| Benzalkonium chloride | 0.01 |
| Carbopol™ 934P | 0.73 |
| Sodium hydroxide | q.s. |
| Purified water | to 100 |

Nedocromil sodium (20 g) and of disodium edetate (2 g) were dissolved in approximately 600 g of purified water. A dispersion of Benzalkonium Chloride Solution 50% USNF (0.808 g) in approximately 200 g of purified water was added and the resulting dispersion made up to 1000 g with purified water and stirred for 30 minutes.

The dispersion was filtered through a glass fibre pre-filter and the first 100 ml discarded. The remainder of the filtered solution was filtered through a pre-sterilised 0.22 µm membrane filter and the filtrate collected.

250 g of the filtrate was added to Carbopol™ 934P (4.15 g) and stirred until the Carbopol™ was fully dispersed.

The pH of the dispersion was adjusted to between pH 5.5 and 5.8 by addition of 2M sodium hydroxide solution. The dispersion was mixed until a homogeneous uniform gel was formed. The gel was made up to 500 g with purified water, remixed and filtered through a 13 µm stainless steel filter.

The viscosity of the solution at 20°C and a shear rate of 50 s⁻¹ was found to lie in the range 420-480 mPa.s.

### Example 6

### Enema Solution

| | |
|---|---|
| Nedocromil Sodium | 0.15 % w/v |
| Nipastat™ | 0.10 |
| Sodium Chloride | 0.812 |
| Purified water | to 100 |

Methyl cellulose or other agents may be added to aid retention of the solution in the bowel.

### Example 7

### Study of 2% Nedocromil Sodium Eye-Drops in the Treatment of Seasonal Allergic Conjunctivitis

32 patients (11 male, 21 female) with ages in the range 4 to 69 (average 25.2) participated in an investigation of the efficacy of an aqueous solution of nedocromil sodium in the treatment of seasonal allergic conjunctivitis. The solution used had the composition given in Example 4. One drop (0.04 ml) was administered per eye four times a day for four weeks.

At the end of the trial, both the patient and the supervising clinician were asked to rate the effectiveness of the treatment, using the following 0-3 scale:
- 0 =: no control of symptoms
- 1 =: slight control
- 2 =: moderate control
- 3 =: full control
In addition the patients were asked whether the eye-drops were an acceptable form of treatment.

For the 23 patients who completed the trial, the results were as follows:

| Rating | Number of Patients | |
|---|---|---|
| | Patient's Assessment | Clinicians Assessment |
| 3 | 10 | 7 |
| 2 | 9 | 9 |
| 1 | 2 | 4 |
| 0 | 1 | 1 |

(One patient failed to record an opinion, and the clinician failed to record an opinion for two patients).

18 of the 23 patients recorded that they found the treatment acceptable.

## Claims

1. A pharmaceutical composition comprising an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, the solution having a pH of from 3.5 to 6.

2. A pharmaceutical composition according to Claim 1, wherein the solution has a pH of from 4 to 5.5.

3. A pharmaceutical composition according to Claim 1 or 2, wherein the concentration of active ingredient in the solution is from 0.1 to 5% w/v.

4. A pharmaceutical composition according to any one of Claims 1 to 3, wherein the solution also contains a preservative.

5. A pharmaceutical composition according to Claim 4, wherein the preservative is chlorbutol.

6. A pharmaceutical composition according to any one of Claims 1 to 5, wherein the solution also contains a viscosity-modifying agent.

7. A pharmaceutical composition according to Claim 6, wherein the solution has a viscosity of from 100 to 10000 mPa.s at a shear rate of 50 s⁻¹.

8. A pharmaceutical composition according to any one of Claims 1 to 7, wherein the active ingredient is nedocromil sodium.

## Claims (Claims for the following Contracting State(s): ES)

1. A process for the preparation of a pharmaceutical composition comprising an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, which process comprises dissolving the active ingredient in purified water optionally containing a preservative, if necessary adding a chelating or sequestering agent and/or a tonicity agent and/or a viscosity-modifying agent, adjusting the pH of the solution to a value within the range 3.5 to 6, optionally diluting the solution and sterilising.

2. A process according to Claim 1, wherein the solution has a pH of from 4 to 5.5.

3. A process according to Claim 1 or 2, wherein the concentration of active ingredient in the solution is from 0.1 to 5% w/v.

4. A process according to any one of Claims 1 to 3, wherein the solution contains a viscosity-modifying agent.

5. A process according to Claim 4, wherein the solution has a viscosity of from 100 to 10000 mPa.s at a shear rate of 50 s⁻¹.

6. A process according to any one of Claims 1 to 5, wherein the active ingredient is nedocromil sodium.
